# EUROPEAN PATENT SPECIFICATION

(11) **EP 1 792 184 B1**
(45) Date of publication and mention of the grant of the patent: **15.07.2009**
(21) Application number: 05762901.6
(22) Date of filing: 27.07.2005
(51) Int. Cl.: G01N 33/558, G01N 33/569

(54) **ASSAY DEVICE & METHOD**
TESTVORRICHTUNG & -VERFAHREN
DISPOSITIF ET METHODE D'ESSAI

(30) Priority: 06.08.2004 GB 0417601
(43) Date of publication of application: 06.06.2007
(73) Proprietor: Inverness Medical Switzerland GmbH, 6300 Zug (CH)
(72) Inventor: ZAK, Krzysztof Wojciech, Bedford, Bedfordshire MK44 3UP (GB); WILLIAMS, David, Bedford, Bedfordshire MK44 3UP (GB)
(74) Representative: Lee, Nicholas John
(86) International application number: PCT/GB2005/002927
(87) International publication number: WO 2006/013329

(56) References cited:
- EP-A- 0 420 021
- EP-A- 0 584 767
- EP-A- 0 895 084
- US-A- 5 415 994
- US-A- 5 536 646
- US-A- 5 773 234
- US-A1- 2001 006 823

## Description

The present invention relates to a device and method for the detection of microbial carbohydrate antigens, and in particular antigens that are characteristic of bacterial organisms, such as, for example, the family Streptococcacae, including Group A and Group B Streptococci.

Immunoassays for the detection of organisms, such as from the family Streptococcae, typically rely on the detection of a specific carbohydrate antigen. Antigenic macromolecules (polysaccharides, polypeptides etc.) which append from the outer surface of bacterial walls are specific to the bacterial species, type group or strain. When these antigenic macromolecules are exposed to the corresponding specific antibodies, the antibodies and the antigens bind together to form a precipitin. However, the antigenic molecules must first be released from the cell wall of the organism before they can be detected. A number of methods exist for carrying out such extraction treatments using for example, nitrous acid, ProNase B enzyme, hot formamide or hot HCL. In the case of nitrous acid, as the nitrous acid contacts the exterior walls of the microorganism, the group antigen is released into solution with its antibody specific reactivity intact

The majority of immunoassay tests that utilise lateral flow technology, for example, utilise nitrous acid for the extraction of the carbohydrate antigen from the organism before contacting the sample containing the extracted antigen with the assay device. Nitrous acid, however, is chemically unstable. It is known only in solution, yet quickly forms nitric acid and nitric oxide in the presence of water. As a result, polysaccharides have conventionally been extracted by the nitrous acid which is formed in a pretreatment step by the reaction between an inorganic nitrate and an aqueous acid, generated immediately prior to antigen extraction. Following the extraction, the solution is then neutralised by the addition of a neutralising agent, such as tris (hydroxymethyl) aminomethane (TRIS) prior to the addition of the resultant solution to the assay device.

Therefore, the extraction of carbohydrate antigen and its subsequent detection would generally be as follows: a swab sample is taken from the back of the throat, for example, and the swab inserted into a vial containing nitrous acid generated by the addition of aqueous solution of sodium nitrite and acetic acid. The swab is subsequently agitated to release the antigens into solution.

Following extraction of the antigen, the solution must be neutralised prior to its addition to a lateral flow or other suitable assay device. In the case of a lateral flow device, the device will typically contain a mobilisable labelled binding reagent capable of binding to the antigen as well as an immobilised binding reagent capable of binding analyte provided downstream from the labelled binding reagent. Thereafter, the antigen may be detected in a conventional manner by formation of an immobilised particulate labelled sandwich binding reagent complex. Such assay devices are disclosed in EP291194.

The method described above, in addition to other prior art methodologies, all require the separate steps of first extracting the antigen by contacting the sample with the precursor nitrite and acid. Both the acid and the nitrite may be contained in solution, or alternatively one of the precursor reactants may be provided in dried form and subsequently contacted with the remaining precursor reactant contained in solution together with the sample. Nevertheless, each of these methodologies require the subsequent step of contacting the sample, following extraction of the antigen, with the lateral flow assay device to detect the desired antigen and which device is provided separately from the sample extraction.

The present invention seeks to alleviate the problems associated with such prior art methodologies and provides a kit and assay device for the detection of antigens or analytes in an organism, wherein the number of treatment steps are substantially reduced and which kit and assay device are therefore particularly easy to use. The test devices according to the invention find particular utility for testing in a nurses or physicians office, a remote or home setting and are therefore particularly useful and suitable for use by non-specialist personnel.

Therefore, according to a first aspect, the invention provides:
a lateral flow assay device for identifying microbial carbohydrate antigens in a biological sample, said device comprising a substrate having
   a) a sample receiving zone,
   b) an extraction zone for receiving the sample from said sample receiving zone and which extraction zone comprises immobilised or otherwise absorbed therein, an acid and a nitrate which, when combined, react to form a nitrous acid which is an extraction reagent for a desired microbial carbohydrate antigen in the sample,
   c) optionally, a neutralising agent capable of bringing the pH of the resulting sample to within the operational pH range of the assay,
   d) a labelled binding reagent specific for said antigen to be detected, and
   e) a detection zone for said labelled binding reagent.

Preferred features of the device of the present invention are set out in the dependent claims herein. Lateral flow assay devices are known in the art and generally comprise a porous material with a reagent containing matrix incorporated therein. The sample is permitted to flow laterally from the sample receiving zone downstream to a reaction or detection zone comprising a capture reagent. In the operation of such devices, a test sample is applied to a sample receiving area or zone on the device and the sample moves or flows through the porous material from the application or sample receiving zone to a reaction or detection zone which includes therein an appropriate capture reagent.

The extraction zone may be provided at or downstream from the sample receiving zone.

In one embodiment of the present invention, the acid and the nitrite are both separately provided in said extraction zone and, when combined by virtue of the movement of the sample through said substrate, react to form said extraction reagent. Therefore, when the solution of sample moves laterally through the assay device, it will contact the acid and the nitrate in succession such that, when they combine, they react to form the extraction reagent which can act on the sample to extract any of the desired antigen present Therefore, the device of the present invention only requires the step of adding the sample to the substrate, thus minimising the number of steps required and the amount of sample and reactant manipulation. The sample to be added to the sample receiving zone may optionally comprise the remaining reactant required to form the extraction reagent if not present in said extraction zone.

The acid is preferably immobilised in the extraction zone of the device by drying, or the like and is provided separate from the nitrite, such that when the sample moves through the lateral flow assay device, it will come into contact with nitrite, both of which will then combine to form the nitrous acid which can then function to extract the antigen from the organism to permit detection.

The acid may be any suitable acid which is capable of reacting with a nitrite to produce nitrous acid, such as a polycarboxylic acid, a polysulfonic acid, a polystyrenesulphoic acid, a polyphosphoric acid, a polyacrylic acid, and a polymethacrylic acid Other such acids are known to those of skill in the art.

The acid however, preferably comprises a non-volatile organic, acid such as any of citric acid, malonic acid, phenylacetic acid, oxalic acid, glycolic acid, chloroacetic acid, tricheroacetic acid, fluoroacetic acid, bomoacetic acid, iodaacetic acid, succinic acid, glutaric acid, adipic acid, pimelic acid, suberic acid, benzoic acid, benzene sulfonic acid, p-toluene sulfonic acid, azelaic acid and sebacic acid. However, a polymeric acid, such as polysulphonic acid is particularly preferred and may be provided on said assay device in the form of a resin. An example of a suitable resin is polystyrene.

The acid may be a polymeric acid and may be immobilised by conjugation to particles. Where the acid is conjugated to a particle, the particle size may, advantageously, be chosen so that it is larger than the pore size of the lateral flow assay such that it is unable to move along the length of the porous carrier with the fluid sample.

Alternatively, the acid may form part of the sample fluid carrying matrix such as a sample receiving wick, or the porous carrier itself. This may be achieved, for example, by sulphonation of the material comprising the particular porous matrix.

Any nitrite which is stable in the dry state and capable of reacting with the immobilised acid to produce nitrous acid may be used, such as an alkali metal nitrite.

The nitrite may be an inorganic nitrite, such as sodium, potassium, lithium, calcium, strontium, barium, and silver nitrite, or an organic nitrite, such as butyl and isoamyl nitrites. Sodium and potassium nitrites are preferred while sodium nitrite is most preferred. The concentration of nitrite and organic acid combined in the extraction reagent can be varied widely depending on the particular compounds used, their water solubility and the suspected amount of antigen present in the test sample.

The sodium nitrite may be added to the device in either an aqueous or dry state.

A neutralising agent may also be provided where necessary to bring the fluid sample after it has contacted the extraction zone to within the operational pH range of the assay. The pH range of the assay may vary and will be dependent upon the nature and type of the capture reagents (for example the particular antibody type and whether it is polyclonal or monoclonal) as well as the antigen to be determined. In some circumstances a neutralising agent may not be required should the pH of the resultant sample mixture fall within the operational pH range of the assay.

However, a neutralising agent is preferred as it allows the pH of the reaction mixture to be optimised which in turn allows for optimisation of the assay sensitivity. A typical pH range may be between pH 6-9. The neutralising agent where present is preferably buffered. Many such neutralising buffers are known in the art, although a preferred one is tris (hydroxymethyl) aminomethane (TRIS).

The location of the neutralising agent, where present, will be determined by the operational pH range of the binding / capture reagents used in the device. Thus, the neutralising agent may be provided downstream from the extraction zone and upstream from a capture reagent. Alternatively, the neutralising agent may be provided downstream from a first capture reagent and upstream from a second capture reagent. Preferably it is provided within the device in the dry state prior to use.

In the invention, a labelled binding reagent specific for the antigen to be detected is located upstream of the detection zone. The detection zone itself may comprise a capture reagent specific for said antigen to be detected, which may be provided in dried or otherwise immobilised form within the detection zone.

The mobilisable labelled binding reagent is preferably located at a position downstream from the optional neutralising reagent. The mobilisable binding reagent may be provided on and/or in a separate porous carrier arranged so as to be in fluidic contact with and upstream from the porous carrier, such as disclosed by US6352862. The separate porous carrier may be macroporous and may be of a different material to that of the porous carrier. According to one embodiment, the porous carrier is nitrocellulose and the macroporous carrier is chosen from one of a glass fibre or a synthetic polymeric material, such as polypropylene.

Alternatively, the mobilisable capture reagent may be provided in the region of the sample receiving wick or the porous carrier.

The binding / capture reagents may be chosen from any species that is capable of forming a binding pair with the carbohydrate antigen and may be, for example, an antibody or fragment thereof, a lectin, boronic acid or boronic acid derivative. The capture reagent may be a specific capture reagent. The antibody may be either polyclonal or monoclonal.

The presence or amount of analyte in the sample may be determined by the detection of the labelled binding reagent present in the detection zone. The labelled binding reagent may be determined visually or by other suitable means.

The label may be chosen from any suitable label, such as an enzyme label or a specific dye such as dyed latex spheres or beads or dye imbibed liposomes. The particular label may also comprise a metal sol, such as a gold sol.

A control zone may also be provided in the device according to the invention, which control zone may be provided at a position at or downstream from the detection zone. The control zone serves to indicate whether the test has been carried out correctly. Typically, the control zone comprises an immobilised capture reagent which is able to capture the labelled binding reagent

In one embodiment of the device of the present invention, the porous material, preferably comprises a porous wick, which can be made or obtained from any bibulous, porous or fibrous material capable of absorbing liquid rapidly. The porosity of the material can be unidirectional (i.e. with pores or fibres running wholly or predominantly parallel to an axis of the member) or multidirectional (omni directional, so that the member has an amorphous sponge-like structure). Porous plastics material, such as polypropylene, polyethylene (preferably of very high molecular weight), polyvinylidene fluoride, ethylene, vinyl acetate, acrylonitrile and polytetrafluoro-ethylene can be used.

In an alternative embodiment, however, the assay device may comprise one or more microfluidic channels, the dimensions of which are such as to permit a liquid sample to move through the one or more channels by capillary action. The one or more capillary channels may be provided in addition to or instead of the one or more porous or absorbent matrices forming part of the device.

It can be advantageous to pre-treat the carrier material with a surface active agent during manufacture, as this can reduce any inherent hydrophobicity in the carrier material and therefore enhance its ability to take up and deliver a moist sample rapidly and efficiently. Porous sample receiving members can also be made from paper or other cellulosic materials, such as nitro-cellulose.

If desired, an absorbent "sink" can be provided at the distal end of the substrate, e.g. the carrier material. The absorbent sink may comprise of, for example, Whatman 3MM^{®} chromatography paper, and should provide sufficient absorptive capacity to allow any unbound labelled capture reagent to wash out of the detection zone.

As an alternative to such a sink, it may be sufficient to provide a length of porous solid phase material which extends beyond the detection zone.

According to a second aspect of the invention, there is provided a kit for identifying carbohydrate antigens in a biological sample, which kit comprises a device of the first aspect of the invention and means for contacting said sample with said sample receiving area.

Also provided by a third aspect of the invention is a method of producing an assay device according to the first aspect of the invention, which method comprises immobilising in a lateral flow assay device having a sample receiving zone, an acid and a nitrate in an extraction zone of said device which, when combined, form a nitrous acid which is an extraction reagent for any carbohydrate antigen in a sample added to said sample receiving zone, optionally providing a neutralisation buffer to neutralise the pH of said extraction reagent subsequent to extraction of said antigen from said sample, said neutralisation buffer being provided downstream of said extraction zone and upstream from a capture/detection reagent immobilised in said assay device, the capture/detection reagent being located at a position upstream from the detection zone and mobilisable.

In a fourth aspect, the invention comprises a method for identifying carbohydrate antigens in a biological sample, which method comprises applying said biological sample to a sample receiving zone of a device of the first aspect of the invention, and monitoring for the presence of said antigen in said detection zone.

The present invention may be more clearly understood from the following exemplary embodiment which is provided by way of example only, with reference to the accompanying drawings wherein;
Fig 1. is an illustration of a device according to the invention.
Fig. 2 is an illustration of the visual results obtained using an assay device of the invention to detect Group A and C Streptococci.

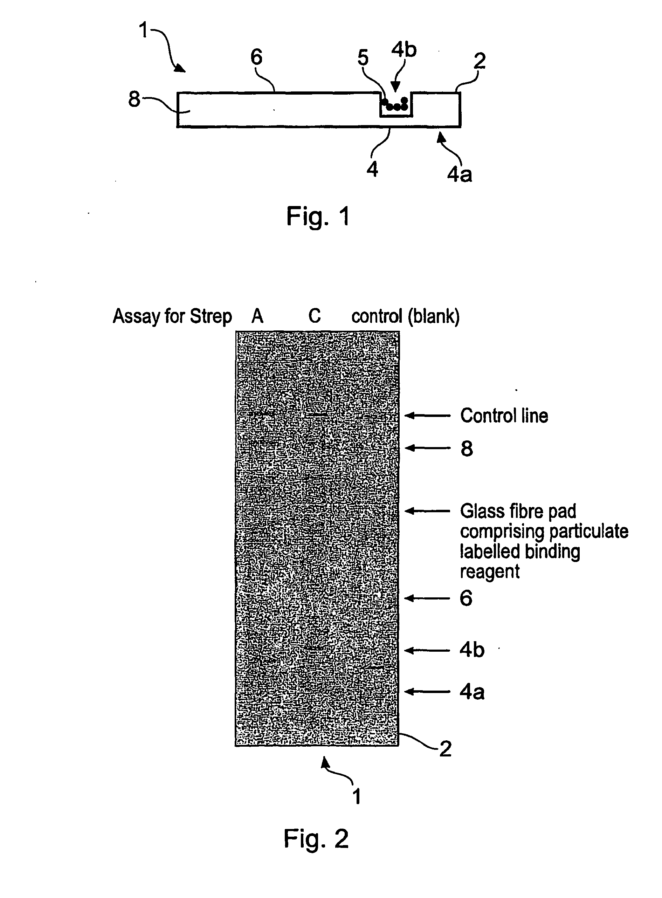

In the device according to the invention as illustrated in Figs.1 to 2, there is provided a lateral flow assay device generally indicated by the reference numeral 1, comprising a sample receiving zone or area 2 and an extraction zone 4 for receiving sample moving laterally through the device 1 in the direction from the sample receiving zone to a detection zone 8. In the extraction zone there is provided in a dried form or otherwise immobilised therein a nitrite 4a and an organic acid 4b. A preferred nitrite is a sodium nitrite whereas the preferred acid is a polysulfonic acid provided in the form of a resin. The polysulfonic acid is provided in a groove or channel 5 cut or etched in the device.

A neutralising buffer 6 is provided downstream of the nitrite and acid and upstream (as defined by the direction of flow in the assay device) of a capture reagent provided in the capture or detection zone 8.

The capture or detection reagent comprises a labelled antibody specific for the antigen to be detected and which can be visualised by virtue of an antigen-antibody complex in the detection/capture zone. Preferably, the antibody is specific for Streptoccal A antigen.

### Experimental

A groove of approx 2mm wide x 3mm deep was made at a distance of 10mm from the near end of a porous sample receiving wick of dimensions 48mm in length x 7mm width x 4mm depth (Filtrona, Richmond Inc, Colonial Heights, Virginia, USA).

250µ 1M sodium nitrite was applied to one end of the wick and allowed to soak in. 200µl of 1.6M TRIS pH 8.5 was subsequently applied to the wick at the top end and allowed to soak in, as indicated in fig. 1. The wick was dried at 50°C overnight. Approximately 20 mg of sulfonic acid resin (MP 70-90 mesh Novabiochem cat. No. 01-64-0432 1.6 mmole /g) was subsequently packed into the groove.

### Preparation of a porous release matrix comprising a mobilisable particulate labelled binding reagent

Anti Strep A carbohydrate antibody (Biotech Inc.) was absorbed onto 400 mm blue latex particles (Duke Scientific, USA) at a concentration of 30 µg/ml (0.5% latex solids) was prepared containing the freeze dried latex particles coated with the antibody. The antibody sensitised latex particles were infused into a glass fibre pad of dimensions 20 mm x 8 mm x 1.2 mm pad having an open structure (Sintair, USA) and freeze dried.

The wick containing the dried extraction reagents was subsequently placed on top of the prepared glass fibre pad.

### Preparation of the porous carrier comprising an immobilised capture reagent

Anti Strep A polyclonal antibody was deposited by means of a microprocessor-controlled microsyringe onto an 8mm wide porous nitrocellulose carrier strip (Schleicher and Schuell based 8µ porosity) at a concentration of 1.9 mg/ml and at a flow rate of 0.8µl/mm.

The antibody was applied in the form of a stripe oriented perpendicular to the flow of liquid sample at a detection zone located downstream from the labelled antibody and the antibody was subsequently blocked with 1% (w/v) polyvinyl alcohol / 3% (w/v) sucrose to immobilise it to the porous carrier.

### Construction of the device

Lateral flow carrier devices were constructed as shown in Figure 1 using the materials as prepared above. A porous sample receiving wick containing the dried extraction reagents was placed on top of a glass pad containing the freeze dried antibody sensitised latex particles coated with anti Strep A polyclonal antibody.

The glass pad was subsequently placed in contact with the nitrocellulose porous carrier such that liquid sample applied to one end of the porous sample receiving wick would be capable of permeating into the glass fibre pad and along the nitrocellulose porous carrier.

A further anti mouse antibody was immobilised at a location downstream from the immobilised capture reagent which functioned as a control zone. Finally an absorbent sink pad was placed in contact with the distal end of the nitrocellulose strip.

A further device was also constructed for the detection of Streptococcus C as described above except the anti Strep A antibodies were replaced by anti-Strep C antibodies.

Samples of Strep A and C were respectively diluted to 10e9 cfu/ml in water. The bacterial suspensions (100µl) were applied to the near end of the device (sodium nitrite end), followed by 1000µl of water (to move the liquid through the device).

As a control the test was repeated except that 100µl of water containing no bacteria was applied to a device. The results of the tests are shown by Figure 2. As may be seen from Figure 2, the presence of blue latex sensitised antibodies may be observed at the detection zone indicating the presence of Strep A and C carbohydrate antigen.

Thus results show that the respective carbohydrate antigens were released from the bacteria by nitrous acid generated by re-hydration of the reagents contained within the device, and that these antigens were then able to be specifically detected in the subsequent immunoassay.

## Claims

1. A lateral flow assay device for identifying microbial carbohydrate antigens in a biological sample, said device comprising a substrate having
a) a sample receiving zone,
b) an extraction zone for receiving the sample from said sample receiving zone and which extraction zone comprises, immobilised or otherwise absorbed therein, an acid and a nitrite which when combined, react to form a nitrous acid which is an extraction reagent for a desired microbial carbohydrate antigen in said sample,
c) optionally a neutralising agent capable of bringing the pH of the resulting sample to within the operational pH range of the assay,
d) a labelled binding reagent specific for said antigen to be detected, and
e) a detection zone for said labelled specific binding reagent,
wherein said labelled binding reagent is mobilisable and located upstream from the detection zone.

2. A device according to claim 1, wherein said antigen to be detected is a Streptococcal antigen.

3. A device according to claim 2, wherein said Streptococcal antigen is Streptococcal A antigen.

4. A device according to any preceding claim, wherein said acid is a sulfonic acid in the form of a resin and said nitrite is sodium nitrite.

5. A device according to any preceding claim, wherein said substrate comprises a lateral flow porous carrier.

6. A device according to claim 5, wherein the porous carrier is a nitrocellulose carrier.

7. A device according to any preceding claim, wherein said substrate comprises microfluidic channels, the dimensions of which are such as to allow a liquid sample move there through by means of capillary action.

8. A device according to any of claims 1 to 7, wherein said labelled binding reagent is provided at a position downstream from the optional neutralising agent.

9. A device according to claim 8, wherein the labelled binding reagent is provided on and/or in a separate porous carrier arranged so as to be in fluidic contact with and upstream from the porous carrier.

10. A device according to claim 9, wherein said separate porous carrier is macroporous and optionally is of a different material to that of the porous carrier.

11. A device according to claim 10, wherein said porous carrier is nitrocellulose and the macroporous carrier is glass fibre or a synthetic polymeric material, such as polypropylene.

12. A device according to any preceding claim, wherein said labelled binding reagent is a labelled antibody.

13. A device according to any preceding claim, where said label is an enzyme label or a specific dye.

14. A device according to any preceding claim, wherein said sample receiving zone comprises a sample receiving wick made of a bibulous, porous or fibrous material capable of absorbing liquid sample.

15. A device according to any preceding claim, wherein said substrate comprises an absorbent sink at the distal end thereof.

16. A device according to any preceding claim, further comprising a control zone at or downstream from the detection zone, which comprises an immobilised capture reagent that is able of capturing the labelled binding reagent.

17. A device according to any preceding claim, wherein the detection zone comprises an immobilised capture reagent capable of capturing the antigen.

18. A kit for identifying carbohydrate antigens in a biological sample, which kit comprises a device according to any preceding claim and means for contacting said sample with said sample receiving area.

19. A method of producing an assay device according to any of claims 1 to 17, which method comprises
immobilising in a lateral flow assay device having a sample receiving zone, an acid and a nitrite in an extraction zone of said device which, when combined, form a nitrous acid which is an extraction reagent for any carbohydrate antigen in a sample added to said sample receiving zone,
optionally providing a neutralisation buffer to neutralise the pH of said extraction reagent subsequent to extraction of said antigen from said sample, said neutralisation buffer being provided downstream of said extraction zone and upstream from a capture/detection reagent in said assay device, the capture/detection reagent being located at a position upstream from the detection zone and mobilisable.

20. A method for identifying carbohydrate antigens in a biological sample, which method comprises applying said biological sample to a sample receiving zone of a device according to any of claims 1 to 17 and monitoring for the presence of said antigen in said detection zone.

## Patentansprüche

1. Lateral-Flow-Assay-Vorrichtung zum Identifizieren von mikrobiellen Kohlehydratantigenen in einer biologischen Probe, wobei die Vorrichtung ein Substrat mit
a) einem probeaufnehmenden Bereich,
b) einem Extraktionsbereich zum Aufnehmen der Probe aus dem probeaufnehmenden Bereich, wobei der Extraktionsbereich, immobilisiert oder anderweitig in diesem absorbiert, eine Säure und ein Nitrit umfasst, welche in Kombination reagieren, um eine salpetrige Säure zu bilden, die ein Extraktionsreagens für ein gewünschtes mikrobielles Kohlehydratantigen in der Probe ist,
c) wahlweise einem Neutralisierungsmittel, das in der Lage ist, den pH-Wert der resultierenden Probe auf innerhalb des Betriebsbereiches des pH-Werts des Assays zu bringen,
d) einem markierten Bindungsreagens, das für das zu detektierende Antigen spezifisch ist, und
e) einem Detektionsbereich für das markierte spezifische Bindungsreagens
umfasst, wobei das markierte Bindungsreagens mobilisierbar und dem Detektionsbereich vorgeschaltet ist.

2. Vorrichtung nach Anspruch 1, wobei das zu detektierende Antigen ein Streptokokken-Antigen ist.

3. Vorrichtung nach Anspruch 2, wobei das Streptokokken-Antigen ein Streptokokken-A-Antigen ist.

4. Vorrichtung nach einem vorherigen Anspruch, wobei die Säure eine Sulfonsäure in Form eines Harzes ist, und wobei das Nitrit Natriumnitrit ist.

5. Vorrichtung nach einem vorherigen Anspruch, wobei das Substrat einen porösen Lateral-Flow-Träger umfasst.

6. Vorrichtung nach Anspruch 5, wobei der poröse Träger ein Nitrocellulose-Träger ist.

7. Vorrichtung nach einem vorherigen Anspruch, wobei das Substrat Mikrofluidkanäle umfasst, deren Abmessungen so gewählt sind, dass sich eine flüssige Probe mittels Kapillarwirkung durch diese hindurch bewegen kann.

8. Vorrichtung nach einem der Ansprüche 1 bis 7, wobei das markierte Bindungsreagens an einer dem wahlweisen Neutralisierungsmittel nachgeschalteten Position vorgesehen ist.

9. Vorrichtung nach Anspruch 8, wobei das markierte Bindungsreagens auf und/oder in einem separaten porösen Träger vorgesehen ist, der angeordnet ist, um mit dem porösen Träger in Fluidverbindung zu stehen und um diesen vorgeschaltet zu sein.

10. Vorrichtung nach Anspruch 9, wobei der separate poröse Träger makroporös und wahlweise aus einem unterschiedlichen Material als der poröse Träger ist.

11. Vorrichtung nach Anspruch 10, wobei der separate poröse Träger Nitrocellulose und der makroporöse Träger ein Glasfasermaterial oder ein synthetisches Polymermaterial, beispielsweise Polypropylen, ist.

12. Vorrichtung nach einem vorherigen Anspruch, wobei das markierte Bindungsreagens ein markierter Antikörper ist.

13. Vorrichtung nach einem vorherigen Anspruch, wobei die Markierung eine Enzymmarkierung oder ein spezifischer Farbstoff ist.

14. Vorrichtung nach einem vorherigen Anspruch, wobei der probeaufnehmenden Bereich einen probeaufnehmenden Docht aus einem trunksüchtigen, porösen oder faserigen Material umfasst, das zur Absorption einer flüssigen Probe in der Lage ist.

15. Vorrichtung nach einem vorherigen Anspruch, wobei das Substrat an seinem distalen Ende eine absorbierende Senke aufweist.

16. Vorrichtung nach einem vorherigen Anspruch, weiters mit einem Kontrollbereich, der beim Detektionsbereich oder diesem nachgeschaltet liegt und ein immobilisiertes Einfangreagens umfasst, das in der Lage ist, das markierte Bindungsreagens einzufangen.

17. Vorrichtung nach einem vorherigen Anspruch, wobei der Detektionsbereich ein immobilisiertes Einfangreagens umfasst, das zum Einfangen des Antigens in der Lage ist.

18. Kit zum Identifizieren von Kohlehydratantigenen in einer biologischen Probe, wobei der Kit eine Vorrichtung nach einem vorherigen Anspruch und Mittel zum Kontaktieren der Probe mit dem probeaufnehmenden Bereich umfasst.

19. Verfahren zum Herstellen einer Assay-Vorrichtung nach einem der Ansprüche 1 bis 17, wobei das Verfahren umfasst:
Immobilisieren in einer Lateral-Flow-Assay-Vorrichtung mit einem probeaufnehmenden Bereich, einer Säure und einem Nitrit in einem Extraktionsbereich der Vorrichtung, wobei die Säure und das Nitrit, wenn kombiniert, eine salpetrige Säure bilden, die ein Extraktionsreagens für jedwedes mikrobielles Kohlehydratantigen in einer Probe ist, die dem probeaufnehmenden Bereich zugeführt wurde,
wahlweises Bereitstellen eines Neutralisierungspuffers zum Neutralisieren des pH-Werts des Extraktionsreagens nach Extraktion des Antigens aus der Probe, wobei der Neutralisierungspuffers dem Extraktionsbereich nachgeschaltet und einem Einfang-/Detektionsreagens in der Assay-Vorrichtung vorgeschaltet ist, wobei das Einfang-/Detektionsreagens an einer dem Detektionsbereich vorgeschalteten Position angeordnet und mobilisierbar ist.

20. Verfahren zum Identifizieren von Kohlehydratantigenen in einer biologischen Probe, wobei das Verfahren das Anwenden der biologischen Probe auf einen probeaufnehmenden Bereich einer Vorrichtung nach einem der Ansprüche 1 bis 17 und das Überwachen der Anwesenheit des Antigens im Detektionsbereich umfasst.

## Revendications

1. Dispositif d'essai à flux latéral pour identifier des antigènes microbiens d'hydrate de carbone dans un échantillon biologique, ledit dispositif comprenant un substrat ayant :
a) une zone de réception d'échantillon,
b) une zone d'extraction pour recevoir l'échantillon de ladite zone de réception d'échantillon et ladite zone d'extraction comprend, immobilisés ou autrement absorbés dans celle-ci, un acide et un nitrite qui, lorsqu'ils sont combinés, réagissent pour former un acide nitreux qui est un réactif d'extraction pour un antigène microbien d'hydrate de carbone souhaité dans ledit échantillon,
c) un agent neutralisant optionnel capable d'amener le pH de l'échantillon résultant à l'intérieur de la plage opérationnelle de pH de l'essai,
d) un réactif de liaison marqué spécifique pour ledit antigène à détecter, et
e) une zone de détection pour ledit réactif de liaison marqué spécifique,
dans lequel ledit réactif de liaison marqué est mobilisable et situé en amont de la zone de détection.

2. Dispositif selon la revendication 1, dans lequel ledit antigène à détecter est un antigène de streptocoque.

3. Dispositif selon la revendication 2, dans lequel ledit antigène de streptocoque est un antigène de streptocoque A.

4. Dispositif selon l'une quelconque des revendications précédentes, dans lequel ledit acide est un acide sulfonique sous la forme d'une résine et ledit nitrite est du nitrite de sodium.

5. Dispositif selon l'une quelconque des revendications précédentes, dans lequel ledit substrat comprend un porteur poreux à flux latéral.

6. Dispositif selon la revendication 5, dans lequel le porteur poreux est un porteur de nitrocellulose.

7. Dispositif selon l'une quelconque des revendications précédentes, dans lequel ledit substrat comprend des canaux micro-fluidiques, dont les dimensions permettent à un échantillon liquide de les traverser par le biais d'une action capillaire.

8. Dispositif selon l'une quelconque des revendications 1 à 7, dans lequel ledit réactif de liaison marqué est fourni à une position en aval de l'agent neutralisant optionnel.

9. Dispositif selon la revendication 8, dans lequel le réactif de liaison marqué est fourni sur et/ou dans un porteur poreux distinct agencé de manière à être en contact fluidique avec le porteur poreux et en amont de celui-ci.

10. Dispositif selon la revendication 9, dans lequel ledit porteur poreux distinct est macroporeux et optionnellement est d'une matière différente de celle du porteur poreux.

11. Dispositif selon la revendication 10,
dans lequel ledit porteur poreux est de la nitrocellulose et le porteur macroporeux est de la fibre de verre ou une matière polymérique synthétique, comme du polypropylène.

12. Dispositif selon l'une quelconque des revendications précédentes, dans lequel ledit réactif de liaison marqué est un anticorps marqué.

13. Dispositif selon l'une quelconque des revendications précédentes, dans lequel ledit marquage est un marquage d'enzyme ou un colorant spécifique.

14. Dispositif selon l'une quelconque des revendications précédentes, dans lequel ladite zone de réception d'échantillon comprend une mèche de réception d'échantillon constituée d'une matière siccative, poreuse ou fibreuse capable d'absorber un échantillon liquide.

15. Dispositif selon l'une quelconque des revendications précédentes, dans lequel ledit substrat comprend un évier absorbant à l'extrémité distale de celui-ci.

16. Dispositif selon l'une quelconque des revendications précédentes, comprenant en outre une zone de commande à la zone de détection ou en aval de celle-ci, qui comprend un réactif de capture immobilisé qui est capable de capturer le réactif de liaison marqué.

17. Dispositif selon l'une quelconque des revendications précédentes, dans lequel la zone de détection comprend un réactif de capture immobilisé capable de capturer l'antigène.

18. Kit pour identifier des antigènes d'hydrate de carbone dans un échantillon biologique, lequel kit comprenant un dispositif selon l'une quelconque des revendications précédentes et des moyens pour mettre ledit échantillon en contact avec ladite zone de réception d'échantillon.

19. Procédé de production d'un dispositif d'essai selon l'une quelconque des revendications 1 à 17, lequel procédé comprenant les étapes consistant à :
immobiliser, dans un dispositif d'essai à flux latéral ayant une zone de réception d'échantillon, un acide et un nitrite dans une zone d'extraction dudit dispositif qui, lorsqu'ils sont combinés, forment un acide nitreux qui est un réactif d'extraction pour tout antigène d'hydrate de carbone dans un échantillon ajouté à ladite zone de réception d'échantillon,
optionnellement fournir un tampon de neutralisation pour neutraliser le pH dudit réactif d'extraction à la suite de l'extraction dudit antigène dudit échantillon, ledit tampon de neutralisation étant fourni en aval de ladite zone d'extraction et en amont d'un réactif de capture/détection dans ledit dispositif d'essai, le réactif de capture/détection étant situé à une position en amont de la zone de détection et mobilisable.

20. Procédé d'identification d'antigènes d'hydrate de carbone dans un échantillon biologique, lequel procédé comprenant l'étape consistant à appliquer ledit échantillon biologique à une zone de réception d'échantillon d'un dispositif selon l'une quelconque des revendications 1 à 17 et l'étape consistant à surveiller la présence dudit antigène dans ladite zone de détection.
